# EUROPÄISCHE PATENTSCHRIFT

(11) **EP 0 882 462 B1**
(45) Veröffentlichungstag und Bekanntmachung des Hinweises auf die Patenterteilung: **21.01.2004**
(21) Anmeldenummer: 98109646.4
(22) Anmeldetag: 27.05.1998
(51) Int. Cl.: A61M 1/36, A61M 1/10

(54) **Herz-Lungen-Maschine mit nichtgeradlinig angeordneten Blutpumpen**
Cardio pulmonary system comprising non-rectilinearly arranged blood pumps
Système cardiopulmonaire avec pompes sanguines disposèes de façon non rectiligne

(30) Priorität: 05.06.1997 DE 19723671
(43) Veröffentlichungstag der Anmeldung: 09.12.1998
(73) Patentinhaber: Stöckert Instrumente GmbH, D-80939 München (DE)
(72) Erfinder: Knott, Erwin, Dr.-Ing., 85586 Poing (DE); Hahn, Andreas, Dr.-Ing., 82054 Sauerlach (DE)
(74) Vertreter: Zangs, Rainer E., Dipl.-Ing.

(56) Entgegenhaltungen:
- EP-A- 0 214 803
- DE-A- 3 815 273
- DE-A- 3 834 952
- DE-C- 19 723 621
- US-A- 4 416 658

## Beschreibung

Die Erfindung betrifft Herz-Lungen-Maschinen mit mehr als zwei Blutpumpen, insbesondere Rollenpumpen.

In der Chirurgie werden Herz-Lungen-Maschinen eingesetzt, um zur Unterstützung oder als Ersatz für den körpereigenen Blutkreislauf einen extrakorporalen Blutkreislauf aufzubauen. Seit der ersten Konzeption vor ca. 40 Jahren ist die Herz-Lungen-Maschine ständig weiterentwickelt und verbessert worden. Dabei wurde die Herz-Lungen-Maschine auf verschiedenste Art und Weise gestaltet, indem die Komponenten, wie Blutpumpen, Oxygenator, Wärmetauscher, Bedienelemente, Stromversorgung, Befestigungselemente usw. unterschiedlich angeordnet wurden. Die verschiedenen Gestaltungen verfolgten das Ziel, die Komponenten effizient zu plazieren und gleichzeitig die Herz-Lungen-Maschine einfach bedienbar zu machen. Im Laufe der Jahre ist eine Vielzahl von Herz-Lungen-Maschinen entwickelt und produziert worden, die aber alle mehr oder weniger voluminös waren, wenn mehrere Blutpumpen eingesetzt werden. Dies trifft auch auf modular aufgebaute Herz-Lungen-Maschinen zu, bei denen die Blutpumpen in Form von Modulen gestaltet sind, die neben der Pumpe auch die Stromversorgung und die Bedienelemente, sowie eine Halterung umfassen.

Aus DE 38 34 952 A1 ist ein extrakorporales Blutzirkulationssystem bekannt, bei dem Blut in einem extrakorporalen Kreislauf mit Hilfe von Blutpumpen gefördert wird. Dabei kommen mehrere Pumpen zum Einsatz, von denen eine Pumpe die Aufgabe einer Hauptblutpumpe übernimmt. Eine Blutverarbeitungsvorrichtung ist aus EP 0 214 803 A bekannt, bei der ebenfalls mehrere Blutpumpen eingesetzt werden. Hier handelt es sich aber nicht um eine Herz-Lungen-Maschine.

Vor diesem Hintergrund besteht das von der Erfindung zu lösende Problem darin, eine Reduzierung der Größe von Herz-Lungen-Maschinen zu erreichen, ohne daß dabei die Funktionalität oder Bedienbarkeit beeinträchtigt wird.

Die Erfindung löst diese Aufgabe, indem bei der erfindungsgemäßen Herz-Lungen-Maschine die drei oder mehr Blutpumpen gemäß Anspruch 1 angeordnet sind. Vorteilhafte Ausgestaltungen sind in den Unteransprüchen beschrieben.

Mit dem Vorschlag, die mehreren Blutpumpen nicht gradlinig anzuordnen, bricht die Erfindung mit einem bei der Fachwelt vorherrschenden Konstruktionsgedanken, wonach die mehreren Blutpumpen stets entlang einer Geraden nebeneinander angeordnet sind. Seit den Anfängen besitzen alle bisher bekannten Herz-Lungen-Maschinen dieses Konstruktionsmerkmal gemeinsam, da bei allen bekannten Modellen, auch den modular aufgebauten Maschinen, die Blutpumpen stets in einer Reihe nebeneinander angeordnet sind. Es handelt sich dabei gewissermaßen um ein Konstruktionsprinzip, das von allen in diesem Bereich tätigen Fachleuten anerkannt und befolgt wurde. Dies geht so weit, daß auch in medizinisch-technischen Lehrbüchern, wie beispielsweise C. Reed und T. Stafford "Cardiopulmonary Bypass", 1985, die aufgereihte Anordnung der Blutpumpen in einer schematischen Darstellung eines extrakorporalen Blutkreislaufs gewählt wird, obwohl dies die zeichnerische Darstellung erschwert.

Die Erfindung schlägt demgegenüber vor, damit die lineare Anordnung aufzugeben und die Blutpumpen um die übrigen Komponenten, d.h. den Oxygenator und/oder Wärmetauscher herum anzuordnen. Damit wird einerseits ein wesentlich kompakterer Aufbau erzielt und das der Erfindung zugrunde liegende Problem gelöst. Andererseits wird der Vorteil erreicht, daß die Schlauchverbindungen zwischen den Blutpumpen und den übrigen Komponenten der Herz-Lungen-Maschine kürzer und damit die körperfremde Oberfläche des extrakorporalen Blutkreislaufs reduziert wird.

Bezugnehmend auf die Zeichnung wird die Erfindung im folgenden anhand eines Ausführungsbeispiels näher beschrieben.

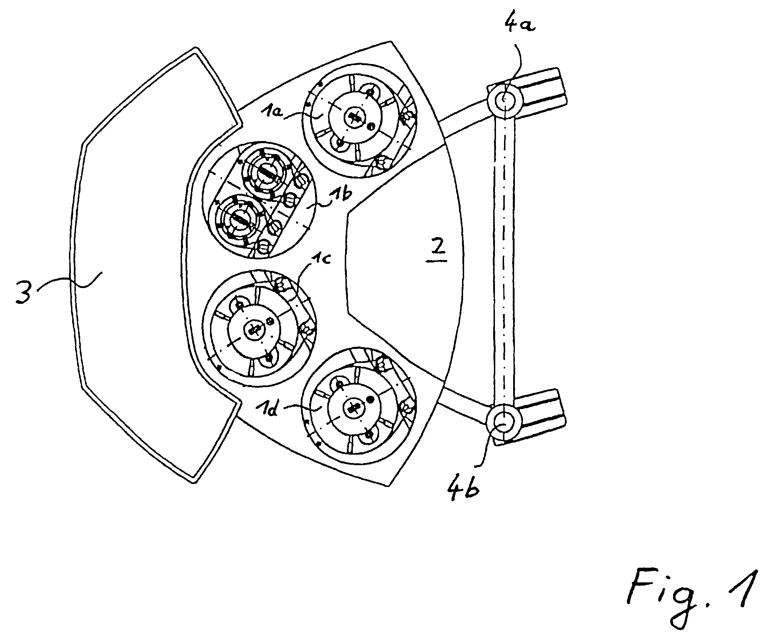

Die Figur 1 zeigt von oben gesehen eine erfindungsgemäße Herz-Lungen-Maschine mit vier Rollenpumpeneinheiten 1a, 1b, 1c und 1d, die nicht geradlinig, sondern um einen Innenbereich 2 herum angeordnet sind. Die Blutpumpen liegen damit entlang einer sich zum Innenbereich 2 hin öffnenden konkaven Linie beliebiger Form. In dem Innenbereich 2 werden ein Oxygenator und/oder ein Wärmetauscher (in der Figur nicht dargestellt), sowie gegebenenfalls andere Komponenten der Herz-Lungen-Maschine plaziert und mit einer, mehreren oder allen Blutpumpen verbunden. Durch die Anordnung der Blutpumpen 1a, 1b, 1c und 1d um den Innenbereich 2 herum wird eine kompakte Anordnung erzielt, die die Größe der Herz-Lungen-Maschine deutlich reduziert. Die Schläuche für den Anschluß der Blutpumpen an den Oxygenator/Wärmetauscher sind gegenüber den herkömmlichen Herz-Lungen-Maschinen verkürzt.

Auf der dem Innenbereich 2 gegenüberliegenden Seite der Blutpumpen ist ein Bedienfeld 3 vorgesehen, in dem mehrere für die Einstellung und Überwachung der Blutpumpen erforderliche Bedienelemente und Kontrollinstrumente angeordnet sind.

Ebenfalls in unmittelbarer Nähe zum Innenbereich 2 und auf der dem Bedienfeld abgewandten Seite der Blutpumpen sind zwei Befestigungsmasten 4a und 4b vorgesehen, die in der Figur 1 nur im Querschnitt zu erkennen sind. An diesen Masten 4a und 4b können der Oxygenator, der Wärmetauscher und andere Komponenten der Herz-Lungen-Maschine befestigt werden.

Die in Figur 1 erkennbaren Pumpenköpfe der Blutpumpen 1a, 1b, 1c und 1d, die bei dem gezeigten Ausführungsbeispiel als Rollenpumpen ausgestaltet sind, sind in der erfindungsgemäßen Herz-Lungen-Maschine vorzugsweise drehbar, so daß der Bereich der Pumpenköpfe, durch den ein in den Pumpenkopf eingelegter Schlauch aus dem Pumpenkopf herausgeführt wird, auf den Innenbereich 2 und damit auf den dort angeordneten Oxygenator/Wärmetauscher ausgerichtet werden kann. Auch diese Maßnahme trägt zu einer Verkürzung der für den Anschluß erforderlichen Schläuche bei.

## Patentansprüche

1. Herz-Lungen-Maschine für die Förderung von Blut in einem extrakorporalen Kreislauf, welche mehr als zwei Blutpumpen (1a, 1b, 1c, 1d) aufweist,
**dadurch gekennzeichnet, dass**
die mehr als zwei Blutpumpen (1a, 1b, 1c, 1d) entlang einer Linie angeordnet sind, die sich konkav zu einem Innenbereich (2) der Herz-Lungen-Maschine öffnet, der für die Anordnung von anderen an die Blutpumpen anschließbaren Komponenten der Herz-Lungen-Maschine vorgesehen ist.

2. Herz-Lungen-Maschine nach Anspruch 1,
**dadurch gekennzeichnet, dass**
die Blutpumpen (1a, 1b, 1c, 1d) drehbare Pumpenköpfe aufweisen.

3. Herz-Lungen-Maschine nach Anspruch 1 oder 2,
**dadurch gekennzeichnet, dass**
auf der von dem Innenbereich (2) der Herz-Lungen-Maschine abgewandten Seite der mehr als zwei Blutpumpen (1a, 1b, 1c, 1d) ein Bedienfeld (3) angeordnet ist.

4. Herz-Lungen-Maschine nach einem der Ansprüche 1 bis 3,
**dadurch gekennzeichnet, dass**
auf der dem Innenbereich (2) der Herz-Lungen-Maschine zugewandten Seite der Blutpumpen zwei Befestigungsmasten (4a , 4b) angeordnet sind.

## Claims

1. Heart-lung machine for transporting blood in an extracorporeal circuit which comprises more than two blood pumps (1a, 1b, 1c, 1d), **characterised in that** the more than two blood pumps (1a, 1b, 1c, 1d) are arranged along a line which opens concavely to an internal region (2) of the heart-lung machine which is provided for the arrangement of other components of the heart-lung machine that can be connected to the blood pumps.

2. Heart-lung machine according to claim 1, **characterised in that** the blood pumps (1a, 1b, 1c, 1d) comprise rotatable pump heads.

3. Heart-lung machine according to claim 1 or 2, **characterised in that** on the side of the more than two blood pumps (1a, 1b, 1c, 1d) facing away from the internal region (2) of the heart-lung machine is arranged a control panel (3).

4. Heart-lung machine according to any of claims 1 to 3, **characterised in that** on the side of the blood pumps facing towards the internal region (2) of the heart-lung machine are arranged two mounting poles (4a, 4b).

## Revendications

1. Machine cardio-pulmonaire pour le pompage de sang, dans un circuit extra-corporel, présentant un nombre de pompes sanguines (1a, 1b, 1c, 1d) supérieur à deux, **caractérisée en ce que** les pompes sanguines (1a, 1b, 1c, 1d) en nombre supérieur à deux sont disposées le long d'une ligne s'ouvrant, de façon concave, en une zone intérieure (2) de la machine cardio-pulmonaire, qui est prévue pour le montage d'autres composants, pouvant être raccordés aux pompes sanguines, de la machine cardio-pulmonaire.

2. Machine cardio-pulmonaire selon la revendication 1, **caractérisée en ce que** les pompes sanguines (1a, 1b, 1c, 1d) présentent des têtes de pompe rotatives.

3. Machine cardio-pulmonaire selon la revendication 1 ou 2, **caractérisée en ce qu'**un panneau de commande (3) est disposé sur la face, opposée à la zone intérieure (2) de la machine cardio-pulmonaire, des pompes sanguines (1a, 1b, 1c, 1d) en nombre supérieur à deux.

4. Machine cardia-pulmonaire selon l'une des revendications 1 à 3, **caractérisée en ce que** deux mâts de fixation (4a, 4b) sont disposés sur la face, tournée vers la zone intérieure (2) de la machine cardio-pulmonaire, des pompes sanguines.
